# EUROPEAN PATENT APPLICATION

(11) **EP 4 385 431 A1**
(43) Date of publication of application: **19.06.2024**
(21) Application number: 22213099.9
(22) Date of filing: 13.12.2022
(51) Int. Cl.: A61B 17/42, A61B 5/00, A61B 90/30, A61B 90/00

(54) **SCAR-LIGHT MANIPULATOR**

(71) Applicant: Universität Zürich, 8006 Zürich (CH)
(72) Inventor: BETSCHART, Cornelia, 8091 Zürich (CH); IMESCH, Patrick, 8091 Zürich (CH); SCHAER, Gabriel, 8091 Zürich (CH)
(74) Representative: Schulz Junghans Patentanwälte PartGmbB

(57) **Abstract**

The invention relates to a uterine transillumination device (1) for detecting scars on an uterus (100), particularly a human uterus, the device comprising at least the following components:
a longitudinal body (1B) extending along a longitudinal axis (z), the longitudinal body (1B) having at least a tip portion (10) for being introduced in the cervix and the uterus cavity, wherein the longitudinal body (1B) further comprises a sheath portion (20) for the tip portion (10), wherein the tip portion (10)
a) is movable along the longitudinal axis (z) inward and outward the sheath portion (20),
b) comprises a first side (10-1) facing along a first radial direction (r₁) away from the longitudinal axis (z) and a second side (10-2) facing toward the opposite direction of the first radial direction (r₁),
c) comprises an illumination assembly (30) arranged on the first side (10-1) of the tip portion (10),
wherein the illumination assembly (30) is configured to emit light radially away from the longitudinal axis (z) along the first radial direction (r₁).

## Description

The invention relates to a device, a system, a method as well as a computer program for transillumination of the uterine wall.

With the rise of cesarean section (c-section), defect scar healing of the uterine wall occurs more frequently. This defect in the anterior uterine wall is called isthmocele, mucocele or niche. Such a niche after c-section has a prevalence of 19% to 84%. For the obstetrical outcome the remaining myometrium over the Niche is of prognostic value. Is the remaining uterine wall too thin (<3mm), the risk of rupture during pregnancy rises for every subsequent pregnancy.

The correction of the uterine scar is a surgical procedure. It is ideally performed with a minimally invasive technique such as laparoscopy that can be combined with or without the use of a surgical robot. In contrast to an open abdominal incision, such as a c-section, the minimal-invasive approach avoids further tissue damage or adhesions and at the same time aims to correct scar problems and improper healing (e.g., scar dehiscence). For an effective correction of the Niche, an optimal visualization of the surgical area that is covered by adhesions is of high importance.

It is an object of the present invention is to provide a device that allows for proper illumination.

The object is achieved by the device having the features of claim 1, the system according to claim 11, the method according to claim 13, the computer program according to claim 14.

Advantageous embodiments are described in the dependent claims.

According to a first aspect of the invention, a uterine transillumination device for detecting scars on a uterus, particularly a human uterus, particularly of the uterine wall facing the abdominal wall, is disclosed, the device comprising at least the following components:
a longitudinal body extending along a longitudinal axis, the longitudinal body having at least a tip portion for being introduced in the cervix and the uterus cavity, wherein the longitudinal body further comprises a sheath portion for sheathing the tip portion, wherein the tip portion
a) is movable along the longitudinal axis inward and outward the sheath portion,
b) comprises a first side facing along a first radial direction away from the longitudinal axis and a second side facing toward the opposite direction of the first radial direction,
c) comprises an illumination assembly arranged on the first side of the tip portion,
wherein the illumination assembly is configured to emit light radially away from the longitudinal axis along the first radial direction.

The device advantageously allows generation of a radial illumination cone that illuminates at least a portion of the uterine cavity wall, where a niche may be located. In contrast to devices known in the art, the illumination profile of the device according to the invention, is aimed in a radial direction and not along the longitudinal axis.

The term "radial illumination" may particularly be understood as sectoral illumination. The sectoral illumination is restricted to an angular sector around the longitudinal axis, particularly wherein the sector may be centered around the first radial direction.

This design of the illumination assembly allows to direct the light from the device in at an angle of approximately 90° ventrally through the anterior vaginal wall during operation, which in turn allows for a better handling and transillumination from the uterus cavity toward the uterine wall.

Particularly, the device is an intra-uterine transillumination device. Niche locations on the uterine wall may be identified by their differing degree of absorption of transilluminated light, such that a niche (typically a zig-zagging dark or gray region on the uterine wall) may be recognized by analyzing a spatial light distribution of the transillumination light. In addition, said transilluminated light distribution allows for identifying thinner and thicker portions of the uterine wall independently of a presence of a niche.

In order for the device being adaptable to different physiologies and morphologies of the female body and in particular the cervix and uterus, the tip portion is movable along the longitudinal axis of the longitudinal body. This allows to adapt the tip portion to varying sizes of the uterine cavity. The tip portion is configured to be inserted into the cavity of the uterus via the cervix. For this reason, a diameter or a circumference of the tip portion is selected accordingly. The tip portion may have a diameter of two to three millimeters which allows passing through the cervix.

Further, the tip portion may be of tubular shape. The sheath portion may be formed as a tube as well, wherein the tube is formed such that the tip portion can be retracted into and moved out of the tube along the longitudinal axis.

The sheath portion may be formed as a guiding tube.

The illumination assembly comprises at least one light source. The term light source particularly refers to a member from which light originates or emanates. Such a member may be a light-generating device, such as an LED, and/or an end portion of an optical guide, e.g. an optical fiber, from which light may emanate, wherein the light may be generated at a different location and guided to the end portion of the optical guide. The illumination assembly may comprise the light-generating device as well as an optical guide.

The term 'first side' particularly refers to a portion on the tip portion that faces radially away from the longitudinal axis and covers an azimuthal angle section, wherein the azimuthal angle range circumferentially extends around the longitudinal axis.

In this context, a cylindrical coordinate system may be associated to the device, wherein a z-axis of the cylindrical coordinate system extends along the longitudinal axis, the radial direction of the cylindrical coordinate system corresponds to the direction pointing radially away from the longitudinal axis, and the angle coordinate of the cylindrical coordinate system corresponds to the azimuthal angle. Particularly, the first radial direction - that extends orthogonally away from the first side - may define an angle of 0° with respect to the coordinate system.

The first side may comprise an azimuthally curved surface portion of the tip portion, which in case the tip portion is of cylindrical shape corresponds to a cylinder shell portion of the tip portion.

The device is particularly configured and designed such that during operation the illumination assembly is not in direct contact with the tissue of the uterine wall, i.e. the illumination assembly, and a section of the tip portion comprising said assembly is held from medical personnel such that said section is not touching any tissue of the uterine wall.

This allows for a reduced heat flow into the tissue during operation, as heat may not be dissipated into the tissue by thermal conduction, but mainly by means of radiation, which is comparably lower than typical thermal conduction.

The illumination assembly may be housed in a transparent housing that grants a distance between the tissue and the illumination assembly.

Furthermore, due to the elongated, longitudinal body, and the tip portion, it is hardly or not possible to bring the illumination assembly, or the first side of the tip portion in direct contact with the uterine wall at the section of the tip portion that comprises the illumination assembly.

According to another embodiment of the invention, the sheath portion and the tip portion are elongated, straight elements that extend symmetrically around the longitudinal axis.

Particularly, the sheath portion and the tip portion comprise a cylindrical shaped section, exhibiting a circular, an elliptical, or oval base area orthogonal to the longitudinal axis. The sheath portion may comprise a hollow cylindrical section, wherein the tip potion may be cylindrical to the extent it is extendible outward the sheath portion. Particularly, the cylinder axis of the sheath and the tip portion may be identical to the longitudinal axis of the device.

This geometry in particular allows to not touch the uterine wall tissue with the illumination assembly.

According to another embodiment of the invention, the tip portion is azimuthally rotatable around the longitudinal axis relative to the sheath portion.

This embodiment allows for adjusting a generated illumination cone or illumination volume in its direction inside the uterus cavity, without the need to rotate the sheath portion as well, leading to a patient-compliant handling improvement as well as an improved flexibility in adjusting the illumination.

For this purpose, the tip portion may bay formed cylindrical and the longitudinal body and/or the sheath portion may be formed as a cylindrical tube, with an inner diameter greater than an outer diameter of the tip portion.

According to another embodiment of the invention, the first radial direction extends orthogonally away from the first side, and wherein the illumination assembly is configured to emit light within, particularly only within an azimuthal angle range, wherein said azimuthal angle range extends relative to the first radial direction, wherein said azimuthal angle range covers azimuthal angles in the range of -110° to 110°, particularly wherein the azimuthal angle of 0° lies on the first radial direction, particularly wherein the angle range extends from -70° to 70°, more particularly the angle range extends from -40° to 40°.

This embodiment particularly relates to the sectoral illumination as described previously, and particularly excludes an illumination profile that would provide a circumferential illumination profile with respect to the longitudinal axis.

This sectoral illumination is restricted to an azimuthal angel range.

This allows for a defined illumination cone extending mainly radially away from the first side such that only a selectable region on the uterine wall may be illuminated.

For example, in case the illumination assembly extends at least in a portion along the longitudinal axis the resulting illumination cone may have the shape of a sector of a cylinder. Thus, the term illumination cone in the context of the current specification may not refer to a cone, but to a differently shaped volume.

The smaller the azimuthal angle range the more concise or focused the illumination volume, which in turn reduces the amount of dissipated power into the uterus, which allows for longer observation times.

According to another embodiment of the invention, the illumination assembly is configured to not to emit light along the longitudinal axis toward the distal end of the longitudinal body and/or toward the second side of the tip portion.

This embodiment further specifies the shape of the illumination volume to be distinct from any device that comprises an illumination assembly for illuminating a volume in extension of the longitudinal axis, i.e. longitudinally with respect the tip portion. These devices are comparably useless for identifying niches in the uterus as the light generally propagates along the wrong direction.

According to another embodiment of the invention, the illumination assembly comprises a plurality of light sources that are configured to be controlled selectively, wherein the light sources are arranged on the first side of the tip portion along the longitudinal axis, particularly wherein the light sources are arranged in a row along the longitudinal direction, such that the illumination assembly extends at least along a portion of the tip portion.

The term 'light sources' is to be understood as elaborated in a previous paragraph of this specification.

This embodiment allows to adjust power dissipated from the illumination assembly into the uterus cavity to be controlled precisely, such that only light sources contributing to the illumination of the uterus cavity may be turned on, wherein light sources that may be covered by the sheath portion or that lie outside the uterus cavity may be turned off.

According to another embodiment of the invention, the illumination assembly is configured to emit light in the wavelength range 590 nm to 1000 nm.

Since heating of the surrounding tissue, e.g. the uterus cavity and uterine wall should be avoided, the selected wavelength range for the illumination assembly allows a greater amount of the emitted light to transilluminate the uterine wall tissue, while keeping the absorption lower as compared to light in a shorter wavelength range, which allows to reduce an energy input into the uterus.

According to another embodiment of the invention, the longitudinal body further comprises a contact portion that has an annular shape and that is at least partially, particularly completely, translucent, wherein the contact portion protrudes radially away from the sheath portion and is configured to be brought in occluding contact to the external orifice of the cervix, particularly wherein the contact is a sealing contact such that the external orifice of the cervix is sealed in a gas tight fashion by the contact portion.

The translucent contact portion allows light to also propagate in regions of the uterus that are close to the entry opening (external orifice) of the uterus, such that niches close to the external orifice may be transilluminated as well.

According to another embodiment of the invention, the longitudinal body further comprises a control portion , wherein the control portion is arranged on a proximal end of the sheath portion and is fixedly connected to the tip portion and thus rotatable with the tip portion in case the tip portion is azimuthally rotatable, wherein the control portion comprises an indicator that is configured to provide a unique visual information regarding an azimuthal orientation of the first side of the tip portion, particularly with respect to the sheath portion.

The indicator allows identification of the orientation of the first side of the tip portion and thus the direction along which light is emitted by the tip portion, during operation, while the tip portion is not visible, such that an orientation may kept constant during operation.

The indicator may be a visual and/or a geometrical indicator.

According to another embodiment of the invention, the indicator may be a tactile indicator, that is configured and designed to be detected by a tactile interaction with the indicator, e.g. by sensing the indicator with a hand or a finger. This embodiment allows to orient or maintain an orientation of the device under a sterile (and thus nontransparent blanket by medical personnel during operation. For this purpose, the indicator may comprise at least one protrusion or at least one recess, particularly wherein said protrusion or recess extends radially away from a surface of the control portion. The protrusion or recess may have a radial extent (height or depth, respectively) in the range of 0.1 mm to 10 mm.

According to another embodiment of the invention, the sheath portion has two handles extending orthogonally away from the longitudinal axis, such that the handles and the sheath portion assume a T-shaped form. The handles are configured to allow manually holding the device during operation.

According to another embodiment of the invention, the indicator is a protrusion or a visual marker, located on the same side as the first side of the tip portion and being indicative of the first radial direction.

According to an alternative embodiment, the indicator may be provided by the handles. However, in case the tip portion is rotatable with respect to the sheath portion, the handles may not be perfectly well-suited as an indicator, as for the medical personnel it requires memorizing a relative orientation of the handles to the first side of the tip portion, rather than having a direct indicator that for example directly points toward the first radial direction, because it rotates with the tip portion.

According to another embodiment of the invention, the uterine transillumination device is configured to adopt at least two states, an extended state in which the tip portion protrudes further out of the sheath portion along the longitudinal axis than in a compact state of the device in which the tip portion is retracted deeper in the sheath portion than in the extended state, i.e. a distance of an end point of the tip portion to the sheath portion is greater in the extended state than in the compact state, wherein the device further comprises a first releasable fixing device, such as a telescope locking member, wherein said first fixing device is configured to adjustably limit how far the tip portion in the extended state protrudes from the sheath portion along the longitudinal axis, and/or wherein the first fixing device is configured to adjustably fixate the tip portion with respect to an azimuthal orientation relative to the sheath portion.

The first fixing device may be comprised by or arranged at the sheath or the control portion.

This embodiment allows for adjusting the uterine transillumination device for the size of the uterus, prior to or during the operation.

Particularly, the first fixing device may not only be configured to limit the extent to which the tip portion protrudes out of the sheath portion in the extended state, but may also be configured to fix the tip portion in the extended state, such that the tip portion neither retracts nor protrudes further when the first fixing device fixes the tip portion.

The first fixing device may be embodied as a turn-lock-assembly or a rotary lock assembly.

According to another embodiment of the invention, the first fixing device comprises a screw, a spring or a locking or a clamping member, configured to repeatedly lock and unlock the tip portion in the adjustable respective extended or retracted state.

According to another embodiment of the invention, the first fixing device is further configured to repeatedly lock and unlock the control portion with the sheath portion in an adjustable azimuthal orientation, such that a user of the device may adjust the azimuthal orientation. According to this embodiment, the first fixing device allows to adjust, lock and unlock the longitudinal extend of the tip portion relative to the sheath portion and also the azimuthal orientation.

According to an alternative embodiment of the invention, the handle portion or the sheath portion comprises a second releasable fixing device, such as a screw, a spring or a locking or a clamping member, configured to repeatedly lock and unlock the control portion with the sheath portion in an adjustable azimuthal orientation, such that a use of the device may adjust the azimuthal orientation.

According to another embodiment of the invention, the device comprises a battery slot configured to house a battery for providing electricity to the illumination assembly, wherein the device further comprises an electrical circuit connecting the battery slot to the illumination assembly.

This allows for a battery-driven device devoid of cables to an external energy source, which in turn allows a more flexible handling of the device during operation.

According to another embodiment of the invention, the device comprises an electronic circuit system configured to control the illumination assembly.

This embodiment allows for an autonomous control of the device, without the need of external devices that may be connected to the device.

According to another embodiment of the invention, the electronic circuit system is configured to adjustably select a section of the illumination assembly to emit light, particularly such that only a section of the illumination assembly of the tip portion that protrudes out of the sheath portion emits light.

The device may further comprise one or more manual control elements, such as a button, a touch-sensitive element, such as a touch-sensitive screen or screen element, e.g. a capacitive or inductive sensor element, a slider and/or a knob element configured to manually adjust the section. Alternatively, or additionally said section may be adjusted automatically by the electronic circuit system.

According to another embodiment of the invention, the electronic circuit system is configured to adjust an illumination intensity, particularly a time-averaged illumination intensity of the light emitted by the illumination assembly.

This embodiment allows for controlling a dissipated energy from the illumination assembly for controlling an amount of heat introduced in the uterus.

The device may further comprise one or more manual control elements, such as a button, a touch-sensitive element, such as a touch sensitive screen, a slider and/or a knob element configured to adjust the illumination intensity. Alternatively, or additionally said illumination intensity may be adjusted automatically by the electronic circuit system.

According to another embodiment of the invention, the electronic circuit system is configured to modulate the illumination intensity of the light emitted by the illumination assembly with a modulation frequency, particularly by means of a pulse-width modulation, particularly wherein the modulation frequency is selectable or adjustable via input data provided to the electronic circuit system, wherein the input data may be selected from the group consisting of: preselected input data, input data determined from an information obtained from a separate data source, input data provided by a user input.

The preselected input data may provide a modulation frequency of 20 Hz.

The separate data source may be a camera of a different device, wherein the modulation frequency is adjusted with respect to a frame rate of the camera.

For user input, the device may further comprise one or more manual control elements, such as a button, a touch-sensitive element, such as a touch sensitive screen, a slider and/or a knob element configured to adjust the modulation frequency.

This embodiment allows for generating an illumination signal that may be computationally separated from another overlaying illumination signal, e.g. from a light source arranged on the other side of the uterine wall.

As the device is a transillumination device, during operation the emitted light from this transillumination device may be seen after passing through the uterine wall, wherein during operation light is shone from both side onto the uterine wall, making it difficult to distinguish transilluminated light from inside the uterus cavity from light that has its origin outside the uterus cavity. Modulating the light emitted by the device according to the invention allows computational separation and processing of the transilluminated light, such that a transilluminated light distribution may be displayed in a false color overlaid to a camera image on a display.

The invention further relates to a second aspect elaborating on the invention according to the first aspect, namely a system comprising the device according to any one of embodiments of the first aspect.

According to the second aspect of the invention, a uterine transillumination system comprises the uterine transillumination device according to any embodiment of the first aspect, wherein the uterine transillumination system further comprises a surgical system configured to be inserted via a surgical access/incision into the abdominal or the pelvic cavity, wherein the surgical system comprises an illumination device for illuminating the uterine wall from the side of the abdominal cavity or the pelvic cavity, a camera for recording images of the uterine wall from the side of the abdominal cavity or the pelvic cavity.

This system allows to identify niches during surgery, as it is configured to record transilluminated light as well as light from the illumination device of the surgical system with the camera, complementing the surgeon's information about the uterine wall as seen from the abdominal or pelvic cavity with the information on a niche location that is comprised a light distribution of the transilluminated light of the transillumination device.

According to another embodiment of the invention, the system comprises a computer, wherein the computer is configured to receive image data comprising information on the recorded images from the camera and wherein the computer is further configured to generate processed image data from the received image data, wherein the processed image data comprise transillumination image information indicative of a spatial light distribution on the uterine wall of light emitted by the transillumination device and propagated through the uterine wall to the pelvic or abdominal cavity.

This transillumination information in essence allows to identify a relative thickness of the uterine wall, which in turn allows for identifying a niche or regions wherein the uterine wall is comparably thin.

According to another embodiment of the invention, the illumination device of the surgical system is configured to emit unmodulated light at constant intensity and wherein during operation the illumination assembly of the transillumination device is configured to emit modulated light at the modulation frequency.

This allows a computational separation of light stemming from the illumination device of the surgical system and the transillumination device, even in case the wavelength ranges of the illumination device of the surgical system and the transillumination device overlap.

According to another embodiment of the invention, a camera frame rate of the camera is higher than the modulation frequency, particularly wherein the camera frame rate is at least 1.5 times higher than the modulation frequency.

This embodiment allows an improved computational separation of the light from the illumination device of the surgical system and the transillumination device.

According to a third aspect of the invention, a method, particularly a computer-implemented method for imaging the uterine wall by using the uterine transillumination system is disclosed, wherein the method executes at least the steps of:
a) selecting, providing or setting a modulation frequency of the illumination assembly,
b) illuminating the uterine wall from inside the uterine cavity with the illumination assembly of the transillumination device,
c) illuminating the uterine wall from outside the uterine cavity, e.g. from the abdominal or pelvic cavity, with the illumination device of the surgical system,
d) recording a plurality of images of the uterine wall with the camera from the outside of the uterus cavity,
e) transmitting the recorded images in form of image data to a computer, particularly the computer of the system,
f) generating by the computer processed image data from the image data, wherein the processed image data comprise transillumination image information configured to generate one or a series of transillumination images indicative of a spatial light distribution on the uterine wall of light emitted by the transillumination device and propagated through the uterine wall to the pelvic or abdominal cavity,
g) displaying the processed image data in form of one or a series of processed images on a display, particularly wherein the one or the series of processed images comprises the one or the series of the transillumination images as an overlay, particularly as a false color overlay, on the recorded images, such that additional optical information on the uterine wall is obtained.

In case the method is embodied as a computer-implemented method, the computer-implemented method may be configured to receive and transmit data to the corresponding components of the system in order to cause the components to execute the corresponding method steps. For this purpose, the computer of the system may be connected to the components of the system, i.e. the illumination device, the transillumination device and the camera, in order to issue instructions causing the components to execute the method steps.

According to a fourth aspect of the invention, a computer program comprises computer program code that when executed on the system according to the second aspect of the invention, causes the system to execute at least the method steps a) to g), particularly the step b) to g) of the method according to the third aspect.

The computer program may be stored on a non-transitory storage medium. The computer program may further be configured to receive user input and to control the transillumination device, the illumination device of the surgical system as well as the camera of the surgical system. For this purpose, the computer program, when executed on the computer and/or a processor, may be configured to address and control communication connections to the components of the system, i.e. the illumination device, the transillumination device and the camera, in order to execute the method steps.

According to fifth aspect of the invention, a computer-readable, non-transitory storage medium having stored thereon the computer program according to the fourth aspect is claimed.

Exemplary definitions relating to a computer-implementation of the method according to the invention are provided in the following.

The terms 'processor' or 'computer', or system thereof, are used herein as ordinary context of the art, such as a general-purpose processor or a micro-processor, RISC processor, or DSP, possibly comprising additional elements such as memory or communication ports. Optionally or additionally, the terms 'processor' or 'computer' or derivatives thereof denote an apparatus that is capable of carrying out a provided or an incorporated computer program and/or is capable of controlling and/or accessing the storage medium and/or other apparatus such as input and output ports. The terms 'processor' or 'computer' denote also a plurality of processors or computers connected, and/or linked and/or otherwise communicating, possibly sharing one or more other resources such as a non-transitory memory.

Use input, such as for example selection or adjustment of the modulation frequency may be obtained by the computer program via appropriate interfaces, e.g. keyboard, mouse or touch-screen. With respect to step a) it is noted that selection may be performed automatically by the computer program, e.g. by obtaining/querying the camera frame rate and adjust the modulation frequency by means of corresponding instructions provided to the transillumination device accordingly.

Particularly, exemplary embodiments are described below in conjunction with the Figures. The Figures are appended to the claims and are accompanied by text explaining individual features of the shown embodiments and aspects of the present invention. Each individual feature shown in the Figures and/or mentioned in said text of the Figures may be incorporated (also in an isolated fashion) into a claim relating to the device according to the present invention.
- Fig. 1: shows a schematic perspective view of the device according to the invention;
- Fig. 2: shows a schematic side view of the device according to the invention;
- Fig. 3: shows a schematic detail of the tip portion of the device according to the invention (side view);
- Fig. 4: shows a schematic detail of the tip portion of the device according to the invention (front view);
- Fig. 5: shows a schematic view of the system according to the invention; and
- Fig. 6: shows a schematic flow chart of the method according to the invention.

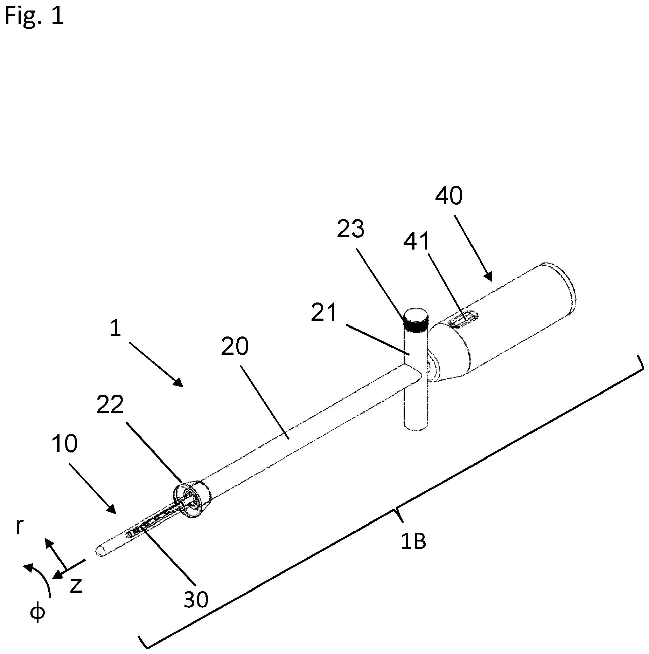

Fig. 1 to Fig. 3 show various views of the transillumination device 1 according an exemplary embodiment of the invention.

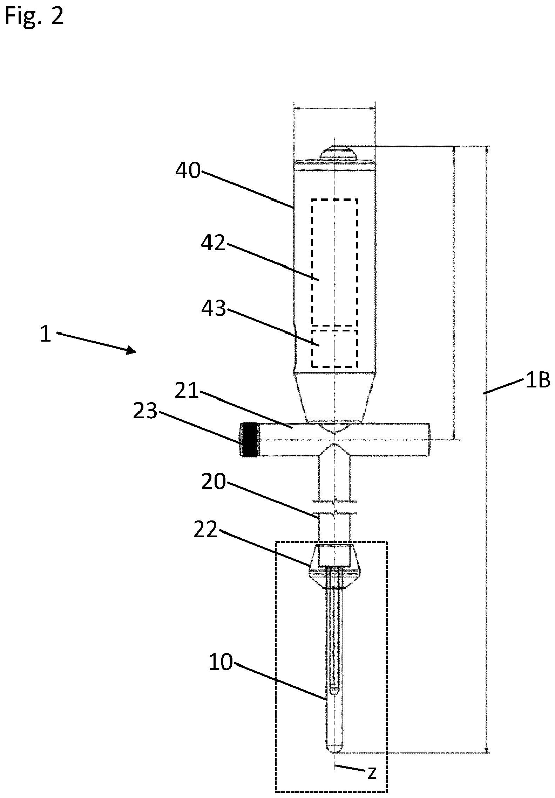

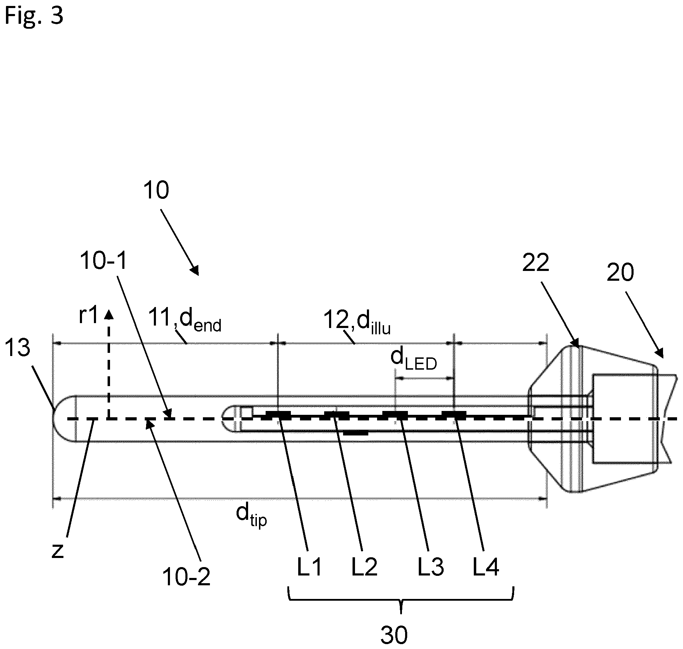

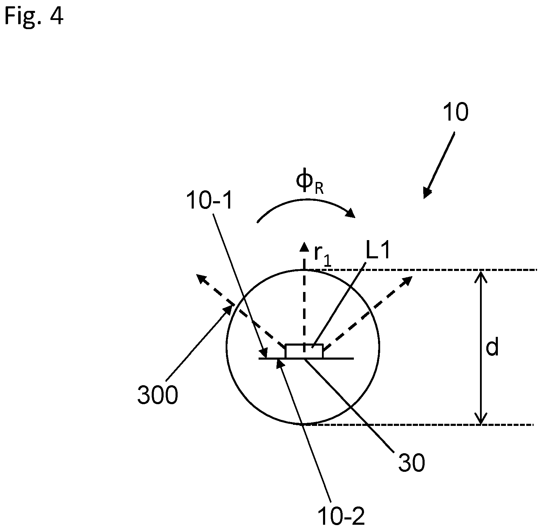

Fig. 1 shows the perspective view of the device according to the invention, wherein Fig. 2 depicts a top view along a radial direction onto the device according to the invention. Figs. 3 and 4 shows a detail of the tip portion of the device according to the invention from different sides.

The following description of the device is provided with reference to Figs. 1 to 4.

The device 1 is a uterine transillumination device 1 for detecting scars on a uterus and comprises several components. As the transillumination device 1, is intended and designed to illuminate the uterus cavity 100 from an inside of the uterus cavity 100, so that during operation emitted light from this transillumination device 1 may be seen or detected after passing through the uterine wall 104. Thus, the transillumination device 1 is configured to be introduced partially into the uterus cavity 100.

For this purpose, the transillumination device 1 comprises a longitudinal body 1B comprising a tip portion 10, a sheath portion 20, and control portion 40. The tip portion 10 is configured to be introduced in the cervix and the uterus cavity 100. Therefore, the tip portion 10 is located at the first (distal) end of the longitudinal body 1B. The tip portion 10 is movable with respect to the longitudinal axis z that extends along the longitudinal body 1B. Particularly, the tip portion 10 is retractable and/or extendable relative to the sheath portion 20, wherein the sheath portion 20 is configured to sheath or at least partially house the tip portion 10.

This allows the transillumination device 1 to be adjusted for different sizes of uteri cavities.

Furthermore, the tip portion 10 has an essentially cylindrical shape, with its cylinder axis extending along the longitudinal axis z. A diameter d of the cylindrical shape may be selected such that the tip portion 10 can be inserted into the cervix, that is typically the diameter ranges from 1 mm to 4 mm. Consequently, the sheath portion 20 may be formed cylindrically or at least may comprise a cylindrical recess to house or partially house the tip portion 10.

The tip portion 10 further comprises a first side 10-1 that faces radially away from the longitudinal axis z in a first radial direction r₁ towards an angular section. The first side 10-1 doesn't need to be flat but may be for example consist of or comprise the half cylindrical surface of the cylinder forming the tip portion 10. Opposite the first side 10-1, a second side 10-2 faces in the other direction.

On the first side 10-1 of the tip portion 10 there is arranged an illumination assembly 30. The illumination assembly 30 is configured to emit light away from the first side 10-1 in a first radial direction r₁ that does not point towards the second side 10-2. In other words, the light cone 300 that may be emitted by the illumination assembly 30 extends into the angular section φ_{R} towards which the first side of tip portion faces. Particularly, the illumination assembly 30 is configured to not to emit light in a direction pointing to the second side 10-2. More particularly, the illumination assembly 30 is not configured to emit light along the longitudinal axis z. Given the geometry of the transillumination device 1 and the anatomy of the female body, particularly the uterus, the emission of light along the radial direction r in a selected angular section, allows to illuminate the uterine wall 104 accurately for transillumination purposes. This is particularly due to the fact that most, if not all, surgeries related to the uterus are performed from a side of the abdominal 103 or the pelvic cavity. That is the transillumination device 1 is configured and adapted to shine light from an inside of the uterus 100 towards a uterine wall 104 that faces toward the pelvic or abdominal cavity 103. A device shining light along the longitudinal axis would for the most part shine light in a that is inaccessible or not accessed during surgeries from the abdominal side. Thus, most of the light would be absorbed by the tissue without being propagating into the desired direction at all contributing to an unnecessary or even harmful heating of the tissue. The transillumination device 1 having the tip portion 10 arranged with the illumination assembly 30 as described emits most photons toward the desired direction.

Turning back to Figures 1 to 4, the tip portion 10 is arranged and configured to be movable along the longitudinal axis z with respect to the sheath portion 20 as elaborated in a previous paragraph, so that a relative extend dtip of the tip portion 10 along the longitudinal axis z that is not covered or enclosed by the sheath portion 20 may be adjusted. This allows to adjust for different uterus cavity sizes, and thus for different anatomies.

The tip portion 10 itself may comprise an end section 11 as well as an illumination section 12. In Fig. 3 it can be seen that the end section 11 extends to the very end 13 of the tip portion 10, wherein the illumination section 12 comprises the illumination assembly 30 and sets forth the end section 11 in a direction towards the sheath portion 20. A distance from a first LED L1 comprised by the illumination assembly to the tip of the tip portion is indicated by d_{end}.

The illumination assembly according to this example comprises four LEDs, L1, L2, L3, L4 that are spaced from each other along the longitudinal axis z with a distance d_{LED}, wherein the LEDs extend over length of dᵢₗₗᵤ, of the illumination assembly along the longitudinal axis z.

The spacing d_{LED} between the LEDs may be in the range of 5mm to 20mm.

The end section 11 may have a length of d_{end} in the range from 0mm to 50mm.

The illumination assembly 30 may extend over a range dᵢₗₗᵤ, from 20mm to 100m.

When the light sources L1, L2, L3, L4 are turned on, the illumination assembly 30 emits light in form of one or more light cones that form the radial illumination cone 300. While for each light cone a principal emission direction may be defined, e.g. by a central axis that extends radially away from the longitudinal axis z, for the plurality of light source, i.e. for the illumination assembly 30, the principal direction of illumination may be defined as an average principal emission direction of the plurality of light cones generated by each light source.

At an end of the sheath portion 20 at a transition to the tip portion 10, a contact portion 22 that has an annular shape is arranged on the transillumination device 1. The contact portion 22 may be formed as a separate contact element and is configured and designed to be brought into occluding contact with the external orifice of the cervix, such as to seal the cervix. The contact portion 22 prevents the sheath portion 20 to be inserted into the cervix, but in essence to blocks the orifice of the cervix. This contact portion is configured and designed to occlude the outer cervical os and to prevent escaping of intraabdominal CO2 that is used to install a pneumoperitoneum for the laparoscopy.

Advantageously, the contact portion 22 is at least partially, particularly completely, translucent, wherein the contact portion protrudes radially away from the sheath portion 20. The translucent contact portion allows light to also propagate in regions of the uterus 100 that are close to the entry opening (external orifice) of the uterus, such that niches close to the external orifice may be transilluminated as well. The contact portion 22 may have a double-conical shape centered on the longitudinal axis, that allows for an improved sealing of the orifice of the cervix. The seal may be formed by a first conus formed by the double-conus. The contact portion 22, particularly the first conus, may overlap or partially overlap with the tip portion 10 along the longitudinal axis z, particularly wherein a second conus of the double-conical shape points along the longitudinal axis z in an opposite direction than the first conus. The contact portion 22 is fixedly attached to the sheath portion 20 and does not move with the tip portion 10.

The transillumination device 1 may be configured to allow the tip portion 10 to be rotated relative to the sheath portion 20. The tip portion 10 may be freely rotatable around the longitudinal axis z, that is the number of turns may be unlimited.

The rotation of the tip portion 10 relative to the sheath portion 20 allows to adjust a principal illumination direction of the illumination assembly 30 in terms of an angular orientation. Said angular orientation is also referred to as azimuthal orientation in the context of the current specification. The principal direction of illumination may be adjusted by turning the tip portion 10 around the longitudinal axis z such that the first side 10-1 of the tip portion 10 and thus the illumination assembly 30 rotates accordingly, and thus faces in an adjusted direction. Accordingly, the principal direction of illumination may be rotated and thus adjusted.

As is depicted for example in Fig. 1 and 2, the transillumination device comprises the control portion 40 that may be rigidly connected to the tip portion 10. In this case the control portion 40 rotates and moves with the tip portion 10 relative to the sheath portion 20. Particularly, when the tip portion is moved inwards the sheath portion, the control portion 40 moves away from the sheath portion and vice versa.

Alternatively, the control portion 40 is fixedly attached to the sheath portion 20.

In this case, the tip portion may also retract and extend into or outwards the control portion 40, for the control portion 40 having a suitable recess on its inside. This allows the control portion 40 to be rigidly attached to the sheath portion 20 or to be integrally formed with the sheath portion 20. The latter embodiment essentially corresponds to a control portion being comprised by the sheath portion.

The control portion 40 may comprise electronic circuits, modules and/or a battery portion 42, a power button.

Further, in order to keep the transillumination device 1 steady during operation and to maintain a constant orientation of the device 1, the device 1 comprises two handles 21 that protrude radially away from the longitudinal axis z, and are fixedly connected to the sheath portion. The two handles may form a "T" together with the sheath portion 20.

The transillumination device 1 further comprises a first fixing device 23, that is configured to lock any longitudinal motion of the tip portion 10 relative to the sheath portion 20.

This allows to adjust a length dₜᵢₚ with which the tip portion 10 protrudes from the sheath portion 20 and thus a distance of the very end of the tip portion to the contact portion.

The first fixing device 23, may comprises a locking mechanism in form of spring lock, a screw or another clamping mechanism. For this purpose, the first fixing device may comprise a turnable knob at an outer end of one of the handles 21 configured to operate the locking mechanism of the first fixing device 23.

The first fixing device 23 may be simultaneously configured to lock the tip portion 10 in terms of an orientation of the illumination assembly 30, i.e. the angular direction in which the first side of the tip portion faces. Alternatively, the transillumination device may comprise a second fixing device configured to lock the tip portion in terms of a rotational motion relative to the sheath portion 20.

The second fixing device (not shown) may be comprised in the second of the two handles. For this purpose, the second fixing device may comprise a turnable knob at an outer end of the second handle configured to operate the locking mechanism of the second fixing device.

Turning to the control portion 40 as depicted e.g. in Fig. 1 or 2, the control portion 40 is configured to control the transillumination device 30. For this purpose, the control portion 40 comprises a battery slot 42 configured to house a battery for providing electricity to the illumination assembly 30. This allows to build the transillumination device 1 as the wireless device, which for example during operation with wired surgical devices is advantageous as handling of the transillumination device is not limited by cables.

The control portion 40 further may comprise a power button for turning the transillumination device 1 on or off, particularly for turning the illumination assembly 30 on and off.

The device 1 comprises an electronic circuit system 43 configured to control the illumination assembly 30.

The electronic circuit system 43 may comprise a microcontroller, and/or microchip or a plurality of microchips, that is configured to adjustably select a section of the illumination assembly 30 to emit light, particularly such that only a section of the illumination assembly of the tip portion that protrudes out of the sheath portion emits light, on the side of the contact portion.

The control portion may further comprise one or more manual control elements, such as a button, a touch-sensitive element, such as a touch-sensitive screen or screen element, e.g. a capacitive or inductive sensor element, a slider and/or a knob element configured to manually adjust the section. Alternatively, or additionally said section may be adjusted automatically by the electronic circuit system.

The electronic circuit system may further be configured to adjust an illumination intensity, particularly a time-averaged illumination intensity of the light emitted by the illumination assembly.

The device 1, particularly the control portion 40 may further comprise one or more manual control elements, such as a button, a touch-sensitive element, such as a touch sensitive screen, a slider and/or a knob element configured to adjust the illumination intensity. Alternatively, or additionally said illumination intensity may be adjusted automatically by the electronic circuit system 43.

The electronic circuit system 43 may be configured to modulate the illumination intensity of the light emitted by the illumination assembly with a modulation frequency, particularly by means of a pulse-width modulation, particularly wherein the modulation frequency is selectable or adjustable via input data provided to the electronic circuit system, wherein the input data may be selected from the group consisting of: preselected input data, input data determined from an information obtained from a separate data source, input data provided by a user input.

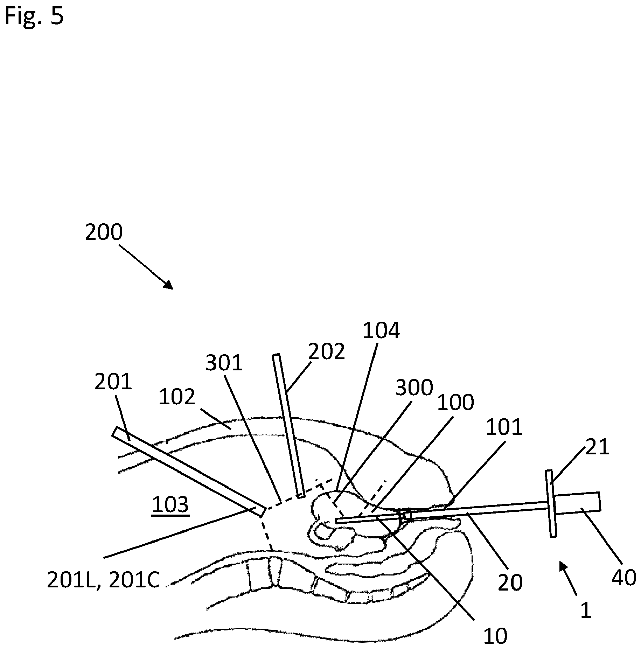

In Fig. 5 a schematic drawing of a system 200 according to an exemplary embodiment of the invention is shown during an operation.

The system 200 comprises the transillumination device 1, a surgical instrument 202, such as a grasping forceps and a laparoscope 201 comprising a light source 201L as well as a camera 201C recording the section in front of its tip.

As can be seen, the laparoscope 201 enters the body via the abdominal wall 102 and shines surgery light 301 from its tip to an outside the uterine wall 104. The surgery light 301 may be approximately white light, covering the spectral regions of blue green and red.

Simultaneously, the transillumination device 1 is inserted via the vagina, the tip portion 10 extending into the uterus 100, with the first side of the tip portion 10, and thus the illumination assembly facing toward the abdominal wall 102. The illumination assembly 30 emits intensity-modulated light 300 on the uterine wall 104 that at least partially propagates through the uterine wall 104. While the light from the illumination assembly 30 may not be seen with the bare eye from the outside of the uterine wall (i.e. from the abdominal side) 104 in case the surgical light 301 is shone from the outside simultaneously, the camera 201C of the laparoscope 201 may record images of the uterine wall 104 and may be sensitive enough to detect the variation in intensity due to the modulated transillumination light 300. A variation of the recorded intensity may be used to computationally separate the light emitted by the illumination assembly and the surgical light emitted by the laparoscope 201.

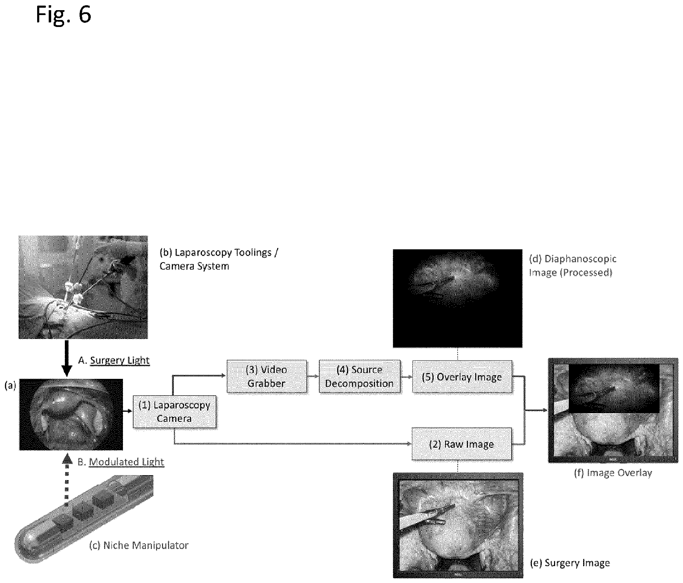

This is schematically shown in Fig. 6. In panel (b) the surgical system 200 for laparoscopy is shown during a minimal invasive surgery.

The laparoscope 201 illuminates the uterine wall 104 from the side of the abdominal wall with surgery light 301 and records (step (1)) images of the uterine wall (panel (a)) with a camera 201C outside of the uterus and transmits said images in form of image data to a computer (not shown). Simultaneously, the transillumination device 1 emits light from the illumination assembly 30 from inside the uterus toward the uterine wall 104. This light is intensity modulated (panel (a)). The recorded images / image data are processed by an image grabber (step (3)) and further processed by means of a source decomposition method (step (4)) that is configured to separate the fraction and areas of transilluminated light (from the illumination assembly) and surgery light (from the laparoscope) based on the modulations in the light of the illumination assembly. Steps (3) and (4) may be executed on the computer. The decomposition method allows to generate processed image data, wherein the processed image data comprise transillumination image information. This information may be used to generate one or a series of transillumination images reflecting an illumination distribution on the uterine wall solely caused by light emitted by the illumination assembly, i.e. where any light contribution of the surgery light is filtered out or at least reduced. The source decomposition method therefore yields at least one image channel, e.g. a first channel comprising the transilluminated light distribution on the uterine wall. The decomposition method may also yield a second channel comprising the surgery light.

The two channels may be combined (step (5)) to an overlay image (panel (f)) and/or displayed as separate images, e.g. a surgery image (panel (e) light from outside the uterus) and a transillumination image (light form inside the uterus; panel (d)). Alternatively, a raw image, as recorded by the camera may be presented as the surgery light image.

The source decomposition may be executed in real time, such that no significant, e.g. less than 0.1 to 0.5 seconds, delay between recording of the images at the camera and display of the images takes place.

The distribution of the transilluminated light may be used to identify areas of scar tissue on the uterine wall.

### Reference signs

| | | | |
|---|---|---|---|
| 1 | transillumination device | r | radial direction |
| 1B | longitudinal body | r₁ | first radial direction |
| 10 | tip portion | z | longitudinal axis |
| 10-1 | first side | φ | angular direction |
| 10-2 | second side | φ_{R} | azimuthal angle range |
| 11 | end section | 100 | uterus |
| 12 | illumination section | 101 | vagina |
| 13 | end of tip | 102 | abdomen/abdominal wall |
| 20 | sheath portion | 103 | abdominal cavity |
| 21 | handles | 104 | uterine wall |
| 22 | contact portion | 200 | surgery system |
| 23 | first fixing device | 201 | surgical tool / laparoscope |
| 30 | illumination assembly | 201L | light |
| 40 | control portion | 201C | camera |
| 41 | indicator of orientation | 202 | grasping forceps |
| 42 | battery slot | 300 | light cone of illumination assembly |
| 43 | electronic circuit system | | |
| L1, L2, L3, L4 | light sources, LEDs | 301 | surgery light from the laparoscope |
| d | diameter of tip portion | | |

## Claims

1. A uterine transillumination device (1) for detecting scars on an uterus (100), particularly a human uterus, the device comprising at least the following components:
a longitudinal body (1B) extending along a longitudinal axis (z), the longitudinal body (1B) having at least a tip portion (10) for being introduced in the cervix and the uterus cavity, wherein the longitudinal body (1B) further comprises a sheath portion (20) for the tip portion (10), wherein the tip portion (10)
a) is movable along the longitudinal axis (z) inward and outward the sheath portion (20),
b) comprises a first side (10-1) facing along a first radial direction (r1) away from the longitudinal axis (z) and a second side (10-2) facing toward the opposite direction of the first radial direction (r₁),
c) comprises an illumination assembly (30) arranged on the first side (10-1) of the tip portion (10),
wherein the illumination assembly (30) is configured to emit light radially away from the longitudinal axis (z) along the first radial direction (r₁).

2. The device (1) according to claim 1, wherein the tip portion (10) is azimuthally rotatable around the longitudinal axis (z) relative to the sheath portion (20).

3. The device (1) according to claim 1 or 2, wherein the first radial direction (r₁) extends orthogonally away from the first side (10-1), and wherein the illumination assembly (30) is configured to emit light within, particularly only within an azimuthal angle range (φ_{R}), wherein said azimuthal angle range (φ_{R}) extends relative to the first radial direction (r₁), wherein said azimuthal angle range (φ_{R}) covers azimuthal angles (φ) in the range of -110° to 110°, particularly wherein the azimuthal angle of 0° lies on the first radial direction (r₁), particularly wherein the angle range extends from -70° to 70°, more particularly the angle range extends from -40° to 40°.

4. The device (1) according to any of the preceding claims, wherein the illumination assembly (30) comprises a plurality of light sources (L1, L2, L3, L4) that are configured to be controlled selectively, wherein the light sources (L1, L2, L3, L4) are arranged on the first side (10-1) of the tip portion (10) along the longitudinal axis (z), particularly wherein the light sources (L1, L2, L3, L4) are arranged in a row along the longitudinal direction (z), such that the illumination assembly (30) extends at least along a portion of the tip portion (10).

5. The device (1) according to any of the previous claims, wherein the illumination assembly (30) is configured to emit light in the wavelength range 590 nm to 1000 nm.

6. The device (1) according to any of the preceding claims, wherein the longitudinal body (1B) further comprises a contact portion (22) that has an annular shape and is at least partially, particularly completely translucent, wherein the contact portion (22) protrudes radially away from the sheath portion (20) and is configured to be brought in occluding contact to the external orifice of the cervix, particularly wherein the contact is a sealing contact such that the external orifice of the cervix is sealed in a gas tight fashion by the contact portion (22).

7. The device (1) according to any of the preceding claims, wherein the longitudinal body (1B) further comprises a control portion (40), wherein the control portion (40) is arranged at a proximal end of the sheath portion (20), particularly wherein the control portion is connected to the tip portion (10) and configured to rotate with the tip portion (10), if the tip portion (10) is azimuthally rotatable with respect to the sheath portion (20), wherein the control portion (40) comprises an indicator (41) that is configured to provide a unique visual information regarding an azimuthal orientation of the first side (10-1) of the tip portion (10), particularly with respect to the sheath portion (20).

8. The device (1) according to any of the preceding claims, wherein the device (1) is configured to adopt at least two states, an extended state in which the tip portion (10) protrudes further out of the sheath portion (20) along the longitudinal axis (z) than in a compact state of the device (1) in which the tip portion (10) is retracted deeper in the sheath portion (20) than in the extended state, wherein the device (1) further comprises a first releasable fixing device (23), wherein said first fixing device (23) is configured to adjustably limit how far the tip portion (10) in the extended state protrudes from the sheath portion (20) along the longitudinal axis (z) and/or wherein the first fixing device (23) is configured to adjustably fixate the tip portion (10) with respect to an azimuthal orientation relative to the sheath portion (20).

9. The device (1) according to any of the preceding claims, wherein the device (1) comprises an electronic circuit system (43) configured to control the illumination assembly (30).

10. The device (1) according to any of the claim 9, wherein the electronic circuit system (43) is configured to modulate the illumination intensity of the light emitted by the illumination assembly (30) with a modulation frequency, particularly by means of a pulse-width modulation, particularly wherein the modulation frequency is selectable or adjustable via input data provided to the electronic circuit system (43), wherein the input data may be selected from the group consisting of: preselected input data, input data determined from an information obtained from a separate data source, input data provided by a user input.

11. A uterine transillumination system (200) comprising the uterine transillumination device (1) according to any of the preceding claims, wherein the uterine transillumination system (200) further comprises a surgical system (201, 202) configured to be inserted via a surgical access/incision into the abdominal or the pelvic cavity (103), wherein the surgical system (201, 202) comprises an illumination device (201L) for illuminating the uterine wall (104) from the side of the abdominal cavity (103) or the pelvic cavity, a camera (201C) for recording images of the uterine wall (104) from the side of the abdominal cavity (103) or the pelvic cavity.

12. The system (200) according to claim 11, wherein the system (200) comprises a computer, wherein the computer is configured to receive image data comprising information on the recorded images from the camera (201C) and wherein the computer is further configured to generate processed image data from the received image data, wherein the processed image data comprises transillumination image information configured to generate one or a series of transillumination images indicative of a spatial light distribution on the uterine wall (104) of light emitted by the transillumination device (1) and propagated through the uterine wall (104) to the pelvic or abdominal cavity (103).

13. A method for imaging the uterine wall (104) executed on the uterine transillumination system (200) according to any of the claims 11 to 12, wherein the method executes at least the steps of:
a) selecting or setting a modulation frequency of the illumination assembly (30),
b) illuminating the uterine wall (104) from inside the uterine cavity (100) with the illumination assembly (30) of the transillumination device (1),
c) illuminating the uterine wall (104) from outside the uterine cavity (100) with the illumination device,
d) recording a series of images of the uterine wall (104) with the camera from the outside of the uterus,
e) transmitting the recorded images in form of image data to a computer, particularly the computer of the system (200),
f) determining by the computer processed image data from the image data, wherein the processed image data comprise transillumination image information being configured to generate one or a series of transillumination images reflecting the uterine wall (104) illuminated only by the transilluminated light from the illumination assembly (30),
g) displaying the processed image data in form of one or a series of processed images on a display, particularly wherein the one or the series of processed images comprises the one or the series of the transillumination images as an overlay, particularly as a false color overlay, on the recorded images, such that additional optical information on the uterine wall (104) is obtained.

14. A computer program comprising computer program code that when executed on the system (200) according to claim 11 or 12, causes the system (200) to execute at least the method steps a) to g), particularly the step b) to g) of the method according to claim 13.

15. A computer-readable, non-transitory storage medium having stored thereon the computer program of claim 14.
